(19) Europäisches Patentamt
European Patent Office
Office européen des brevets

(11) **EP 4 722 266 A1**

(12) **EUROPEAN PATENT APPLICATION**
published in accordance with Art. 153(4) EPC

(43) Date of publication:
08.04.2026 Bulletin 2026/15

(21) Application number: 24815579.8

(22) Date of filing: 30.05.2024

(51) International Patent Classification (IPC):
$C08F\ 290/06$ (2006.01)    $A61C\ 13/15$ (2006.01)
$B29C\ 64/135$ (2017.01)    $B29C\ 64/314$ (2017.01)
$B33Y\ 10/00$ (2015.01)    $B33Y\ 70/00$ (2020.01)
$B33Y\ 80/00$ (2015.01)

(52) Cooperative Patent Classification (CPC):
A61C 19/003; B29C 64/135; B29C 64/314;
B33Y 10/00; B33Y 70/00; B33Y 80/00;
C08F 290/06

(86) International application number:
PCT/JP2024/019910

(87) International publication number:
WO 2024/248099 (05.12.2024 Gazette 2024/49)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
GE KH MA MD TN

(30) Priority: 31.05.2023 JP 2023089590

(71) Applicant: Kuraray Noritake Dental Inc.
Kurashiki-shi, Okayama 710-0801 (JP)

(72) Inventors:
• SUZUKI, Kenji
  Tainai-shi, Niigata 959-2653 (JP)
• ISHIBASHI, Misaki
  Tainai-shi, Niigata 959-2653 (JP)
• NAKAKURA, Nonoka
  Tainai-shi, Niigata 959-2653 (JP)

(74) Representative: D Young & Co LLP
3 Noble Street
London EC2V 7BQ (GB)

(54) **STEREOLITHOGRAPHIC RESIN COMPOSITION**

(57) The present invention provides a stereolithographic resin composition that exhibits excellent strength, toughness, water resistance, and impact resistance in the shaped article. The present invention relates to a stereolithographic resin composition comprising a polyfunctional (meth)acrylic polymerizable compound (A) with a molecular weight of less than 1,000, a polyfunctional (meth)acrylic polymerizable compound (B) with a molecular weight of 1,000 or more, a monofunctional (meth)acrylic polymerizable compound (C) that does not comprise a hydroxyl group and comprises a cyclic ether structure, and a photopolymerization initiator (D).

EP 4 722 266 A1

**Description**

TECHNICAL FIELD

The present invention relates to stereolithographic resin compositions. More specifically, the present invention makes it possible to obtain a three-dimensional shaped article that exhibits excellent strength, toughness, water resistance, and impact resistance. The invention is particularly suited for dental occlusal splints, denture base materials, and appliances for treating sleep apnea, with optimum suitability for soft dental occlusal splints.

BACKGROUND ART

Various proposals have been made concerning stereolithography, a method that produces three-dimensional shaped articles through a repeated procedure whereby liquid photocurable resin is cured into a thin layer under controlled application of necessary amounts of light energy, and another thin layer is cured on the cured layer under controlled application of light after supplying another portion of the liquid photocurable resin onto the previously formed cured layer.

Vat stereolithography is a technique typically used for optical fabrication of three-dimensional shaped articles. In this technique, a liquid photocurable resin composition is placed in a vat, and a computer-controlled ultraviolet laser is selectively applied to the surface of the liquid resin composition to cure it to a predetermined thickness, forming a cured layer with the desired pattern. Continuously, another cured layer is formed on the cured layer by applying an ultraviolet laser in the same manner to the liquid photocurable resin composition supplied onto the previously cured layer in an amount necessary to form a single layer. This layering process is repeated until it produces the final three-dimensional shaped article. In recent years, this technique has attracted great interest because it enables easy and precision production of the desired three-dimensional shaped article in a relatively short time period, even when the article has a very complex shape.

Three-dimensional shaped articles created through stereolithography are expanding their applications from mere concept models to test models, prototypes, and final products. The field of dental materials is thought to greatly benefit from stereolithography because dental occlusal splints, denture bases, and mouthpiece-like materials used for treating sleep disorders (for example, for preventing bruxism or treating sleep apnea) require shapes that vary from patient to patient, aside from being complex in shape.

Some dental occlusal splints are fitted to adjust tooth alignment or jaw position as in so-called orthodontic mouthpieces or aligners while others are attached to teeth to reduce tooth wear due to clenching. Mouthguards are another type of splints that are worn in the mouth to protect the stomatognathic system and the brain by reducing injuries caused when large external forces are applied to teeth and jawbones during sports activities in contact sports. In orthodontics, the use of these devices has gained wide popularity over the last years due to their favorable aesthetics and convenient removability.

Denture base materials are materials used for the gum as a part of a denture attached to replace missing teeth. The demand for dentures has rapidly increased in recent years because of increasing ageing populations.

Appliances for the treatment of sleep apnea include appliances (oral appliances, or OA) attached to teeth during sleep for the treatment of obstructive sleep apnea syndrome (OSAS), and its use has been rapidly increasing.

Common requirements for dental occlusal splints, denture base materials, and OA include strength, toughness, water resistance, and impact resistance. Low strength creates discomfort by increasing flexure or deformation during wear, whereas a loss of toughness necessitates frequent replacement as it increases susceptibility to breakage from biting forces or deformation during wear. A loss of water resistance causes a reduction of mechanical properties during use, making the appliance practically useless by making the appliance easily deformable or breakable while being worn. Furthermore, should the appliance be accidentally dropped during placement, removal, or cleaning, the appliance can easily break if it has low impact resistance.

Another consideration is that fabrication of dental occlusal splints, denture base materials, and appliances for the treatment of sleep apnea typically requires taking an impression of the oral cavity. This is seen as a problem because the procedure involves discomfort, and places a burden on patients, in addition to requiring high technical skills. Recent advances in digital technology have led to approaches that make use of an intraoral optical scan for taking an oral impression, and there have been attempts to apply stereolithography techniques for shaping. For fabrication, a stereolithographic resin composition is used. As a rule, resin compositions that develop strength tend to be more brittle, and attempts to impart toughness (flexibility) while maintaining strength often result in a flexible molecular structure, which encourages moisture penetration. Indeed, it is difficult to satisfy all of strength, toughness, water resistance, and impact resistance.

Against this background, techniques are proposed that enable stereolithographic fabrication of a product having excellent strength, toughness, and impact resistance.
For example, Patent Literature 1 discloses a stereolithographic resin composition in which a urethane acrylate oligomer with a polyester backbone, a urethane acrylate oligomer with a polyether backbone, and a specific di(meth)acrylic acid ester compound with a dioxane backbone are contained as essential components.

CITATION LIST

Patent Literature

Patent Literature 1: WO2020/136919

SUMMARY OF INVENTION

Technical Problem

However, upon investigation by the present inventors, it was found that while the cured product of the stereolithographic resin composition described in Patent Literature 1 exhibits properties that are to some extent superior in strength, toughness, impact resistance, and fatigue characteristics, Patent Literature 1 makes no mention of water resistance, and has room for improvement in water resistance.
It is accordingly an object of the present invention to provide a stereolithographic resin composition that exhibits excellent strength, toughness, water resistance, and impact resistance.

Solution to Problem

The present invention includes the following.

[1] A stereolithographic resin composition comprising a polyfunctional (meth)acrylic polymerizable compound (A) with a molecular weight of less than 1,000, a polyfunctional (meth)acrylic polymerizable compound (B) with a molecular weight of 1,000 or more, a monofunctional (meth)acrylic polymerizable compound (C) that does not comprise a hydroxyl group and comprises a cyclic ether structure, and a photopolymerization initiator (D).
[2] The stereolithographic resin composition according to [1], wherein the polyfunctional (meth)acrylic polymerizable compound (A) is a (meth)acrylate that contains no polymer structure within a single molecule.
[3] The stereolithographic resin composition according to [1] or [2], wherein the polyfunctional (meth)acrylic polymerizable compound (A) comprises a polyfunctional urethanized (meth)acrylic polymerizable compound (A)-I containing a urethane bond.
[4] The stereolithographic resin composition according to any one of [1] to [3], wherein the polyfunctional (meth)acrylic polymerizable compound (B) comprises a polyfunctional urethanized (meth)acrylic polymerizable compound (B)-I containing a urethane bond.
[5] The stereolithographic resin composition according to [4], wherein the polyfunctional urethanized (meth)acrylic polymerizable compound (B)-I is a (meth)acrylate that comprises, within a single molecule, at least one structure selected from the group consisting of a polyester, a polycarbonate, a polyurethane, a polyether, a poly-conjugated diene, and a hydrogenated poly-conjugated diene.
[6] The stereolithographic resin composition according to [5], wherein the polyfunctional urethanized (meth)acrylic polymerizable compound (B)-I is a (meth)acrylate that comprises, within a single molecule, at least one polyol moiety selected from the group consisting of a polyester, a polycarbonate, a polyurethane, a polyether, a poly-conjugated diene, and a hydrogenated poly-conjugated diene each having a structure derived from a C4 to C18 aliphatic chain diol unit having a branched structure.
[7] The stereolithographic resin composition according to any one of [1] to [6], wherein the cyclic ether structure of the monofunctional (meth)acrylic polymerizable compound (C) is a dioxane.
[8] The stereolithographic resin composition according to any one of [1] to [7], wherein the monofunctional (meth)acrylic polymerizable compound (C) is a mono(meth)acrylate.
[9] The stereolithographic resin composition according to any one of [1] to [7], wherein the monofunctional (meth)acrylic polymerizable compound (C) is at least one selected from the group consisting of (2-methyl-2-ethyl-1,3-dioxolan-4-yl)methyl (meth)acrylate, (2-methyl-1,3-dioxolan-4-yl)methyl (meth)acrylate, (2-ethyl-1,3-dioxolan-4-yl)methyl (meth)acrylate, 2-((2-methyl-1,3-dioxolan-4-yl)methoxy)ethyl (meth)acrylate, 2-((2-ethyl-1,3-dioxolan-4-yl)methoxy)ethyl (meth)acrylate, and cyclic trimethylolpropane formal (meth)acrylate.
[10] A dental material comprising a shaped article of the stereolithographic resin composition of any one of [1] to [9].
[11] A denture base material comprising a shaped article of the stereolithographic resin composition of any one of [1] to [9].
[12] A dental occlusal splint comprising a shaped article of the stereolithographic resin composition of any one of [1] to [9].

[13] A material for treating sleep disorder, comprising a shaped article of the stereolithographic resin composition of any one of [1] to [9].

[14] A method for stereolithographically producing a three-dimensional shaped article with the stereolithographic resin composition of any one of [1] to [9].

Advantageous Effects of Invention

A stereolithographic resin composition of the present invention exhibits excellent strength, toughness, water resistance, and impact resistance in the shaped article. This makes a stereolithographic resin composition of the present invention suited for dental occlusal splints and denture base materials, and as a material used for appliances for treating sleep apnea.

DESCRIPTION OF EMBODIMENTS

The following describes the present invention in detail through embodiments.

In this specification, the upper limits and lower limits of numeric ranges (for example, ranges of contents of components, ranges of values calculated from components, and ranges of physical properties) can be appropriately combined. For example, in this specification, the lower and upper limits of numeric ranges presented in a stepwise fashion may be combined independently. As an example, in a statement such as "preferably 0.2 to 8.0 mass%, more preferably 0.5 to 5.0 mass%" for the same parameter, it is possible to combine the preferred lower limit (0.2 mass%) with the more preferred upper limit (5.0 mass%) to form a range of "0.2 to 5.0 mass%", or, alternatively, "0.5 to 8.0 mass%."

Concerning numeric ranges, it is also possible to define only the lower limit without specifying the upper limit, for example, such as "12 mass% or more" or "12.5 mass% or more", based on a statement "more preferably 12 to 28 mass%, even more preferably 12.5 to 25 mass%". Similarly, it is possible to define only the upper limit without specifying the lower limit, such as "28 mass% or less" or "25 mass% or less."

Unless otherwise specified, a numeric range stated simply as "30 to 70" represents the range of 30 or more and 70 or less. Furthermore, for example, when a numeric range is "25 mass% or more and 75 mass% or less", the boundary values of the numeric range may be chosen from any of "more than 25 mass%", "25 mass% or more", "75 mass% or less", and "less than 75 mass%."

Similarly, for example, from statements presented for the same parameter such as "more preferably 0.05 parts or more by mass, even more preferably 0.1 parts or more by mass" and "more preferably 15 parts or less by mass, even more preferably 10 parts or less by mass", it is possible to combine the more preferred lower limit (0.05 parts or more by mass) with the even more preferred upper limit (10 parts or less by mass) to form the range of "0.05 parts or more by mass and 10 parts or less by mass." Furthermore, as with the foregoing, it is also possible to specify only the lower limit as "0.05 parts or more by mass" or "0.1 parts or more by mass", and, similarly, only the upper limit as "15 parts or less by mass" or "10 parts or less by mass."

The numeric ranges described above are merely examples using mass percentages and parts by mass, and the same applies to molecular weight and the like.

As used herein, the term "polymerizable compound" refers to a polymerizable compound polymerized by the photopolymerization initiator (D) described below.

As used herein, the term "(meth)acryl" is intended to be inclusive of both methacryl and acryl, and the same applies to similar expressions such as "(meth)acrylate", "(meth)acrylic acid ester", "(meth)acrylamide", and "(meth)acryloyloxy."

As used herein, the term "(meth)acrylic polymerizable compound" is intended to be inclusive of both polymerizable compounds having a (meth)acryloxy group as a polymerizable group, and polymerizable compounds having a (meth)acrylamide group as a polymerizable group.

As used herein, "polyfunctional" means that the number of polymerizable groups exceeds one.

As used herein, "molecular weight" refers to a single value calculated from atomic weights when an oligomer or polymer structure is absent. When an oligomer or polymer structure with two or more repeating units is present, the term "molecular weight" refers to, unless otherwise specified, the weight-average molecular weight determined by gel permeation chromatography (GPC), expressed in terms of polystyrene equivalents.

A stereolithographic resin composition of the present invention comprises a polyfunctional (meth)acrylic polymerizable compound (A) with a molecular weight of less than 1,000, a polyfunctional (meth)acrylic polymerizable compound (B) with a molecular weight of 1,000 or more, a monofunctional (meth)acrylic polymerizable compound (C) that does not comprise a hydroxyl group and comprises a cyclic ether structure, and a photopolymerization initiator (D).

[Polyfunctional (Meth)Acrylic Polymerizable Compound (A) with Molecular Weight of Less Than 1,000]

In a stereolithographic resin composition of the present invention, the polyfunctional (meth)acrylic polymerizable compound (A) with a molecular weight of less than 1,000 (hereinafter, also referred to simply as "polyfunctional (meth) acrylic polymerizable compound (A)") is used to impart shapeability to the stereolithographic resin composition and to impart strength and toughness to the shaped article.

In view of superior water resistance, the polyfunctional (meth)acrylic polymerizable compound (A) with a molecular weight of less than 1,000 is preferably one that does not contain a polymer structure within a single molecule, more preferably a (meth)acrylate that does not contain a polymer structure within a single molecule. The absence of a polymer structure leads to reduced content of repeating units of polar functional groups per molecule, which tends to improve water resistance.

Examples of the polymer structure include polyester, polycarbonate, polyurethane, polyether, polyamide, polyimide, polyarylate, and polyacrylate.

Examples of polyfunctional (meth)acrylic polymerizable compound (A) include polyfunctional urethanized (meth)acrylic polymerizable compound (A)-I containing a urethane bond (hereinafter, also referred to simply as "polyfunctional urethanized (meth)acrylic polymerizable compound (A)-I"), and polyfunctional (meth)acrylic polymerizable compound (A)-II containing no urethane bond (hereinafter, also referred to simply as "polyfunctional (meth)acrylic polymerizable compound (A)-II"). In view of superior toughness in the shaped article, it is preferable to comprise a polyfunctional urethanized (meth)acrylic polymerizable compound (A)-I. More preferably, the polyfunctional urethanized (meth)acrylic polymerizable compound (A)-I is a (meth)acrylate that does not contain a polymer structure within a single molecule.

As an example, the polyfunctional urethanized (meth)acrylic polymerizable compound (A)-I can be synthesized with ease by an addition reaction of an isocyanate group-containing compound having an alkylene backbone or phenylene backbone with a (meth)acrylic compound having a hydroxyl group (-OH).

The compound having an isocyanate group, and the (meth)acrylic compound having a hydroxyl group (-OH) may be compounds identical or similar to those exemplified below in the production of polyfunctional urethanized (meth)acrylic polymerizable compound (B)-I.

The addition reaction may be performed using known methods and conditions, and is not particularly limited.

Examples of the polyfunctional urethanized (meth)acrylic polymerizable compound (A)-I include polyfunctional urethanized (meth)acrylates such as 2,2,4-trimethylhexamethylenebis(2-carbamoyloxyethyl)dimethacrylate (commonly known as UDMA), 2,4-tolylenebis(2-carbamoyloxyethyl)di(meth)acrylate, N,N-(2,2,4-trimethylhexamethylene)bis[2-(aminocarboxy)propane-1,3-diol]tetra(meth)acrylate, bishydroxyethyl methacrylate-isophorone diurethane, and 2,4-tolylenebis(2-carbamoyloxyethyl)dimethacrylate. Other examples of the polyfunctional urethanized (meth)acrylic polymerizable compound (A)-I include phenyl glycidyl ether (meth)acrylate hexamethylene diisocyanate urethane prepolymer, pentaerythritol tri(meth)acrylate hexamethylene diisocyanate urethane prepolymer, pentaerythritol tri(meth)acrylate toluene diisocyanate urethane prepolymer, and pentaerythritol tri(meth)acrylate isophorone diisocyanate urethane prepolymer. These may be used alone, or two or more thereof may be used in combination. In view of the strength and toughness of the shaped article, preferred among these are 2,2,4-trimethylhexamethylenebis(2-carbamoyloxyethyl)dimethacrylate, and phenyl glycidyl ether acrylate hexamethylene diisocyanate urethane prepolymer, more preferably 2,2,4-trimethylhexamethylenebis(2-carbamoyloxyethyl)dimethacrylate.

In view of even superior strength and toughness in the shaped article, the content of the polyfunctional urethanized (meth)acrylic polymerizable compound (A)-I in polyfunctional (meth)acrylic polymerizable compound (A) is preferably 20 mass% or more, more preferably 40 mass% or more, even more preferably 60 mass% or more, most preferably 80 mass% or more in total 100 mass% of the polyfunctional (meth)acrylic polymerizable compound (A). The content of the polyfunctional urethanized (meth)acrylic polymerizable compound (A)-I in polyfunctional (meth)acrylic polymerizable compound (A) may be 100 mass%.

The polyfunctional (meth)acrylic polymerizable compound (A)-I includes bifunctional (meth)acrylic polymerizable compounds, and tri- and higher-functional (meth)acrylic polymerizable compounds. However, in view of the toughness and impact resistance of the shaped article, preferred are bifunctional (meth)acrylic polymerizable compounds.

The polyfunctional (meth)acrylic polymerizable compound (A)-II includes bifunctional (meth)acrylic polymerizable compounds, and tri- and higher-functional (meth)acrylic polymerizable compounds. However, in view of the toughness and impact resistance of the shaped article, preferred are bifunctional (meth)acrylic polymerizable compounds.

Examples of the polyfunctional (meth)acrylic polymerizable compound (A)-II include 2,2-bis((meth)acryloyloxyphenyl)propane, 2,2-bis(4-(meth)acryloyloxyethoxyphenyl)propane, 2,2-bis(4-(meth)acryloyloxydiethoxyphenyl)propane, 2,2-bis(4-(meth)acryloyloxypropoxyphenyl)propane, 2,2-bis[4-(2-hydroxy-3-methacryloyloxypropoxy)phenyl]propane (commonly known as Bis-GMA), 2,2-bis(4-(meth)acryloyloxytetraethoxyphenyl)propane, 2,2-bis(4-(meth)acryloyloxypentaethoxyphenyl)propane, 2-(4-(meth)acryloyloxydiethoxyphenyl)-2-(4-(meth)acryloyloxyethoxyphenyl)propane, 2-(4-(meth)acryloyloxydiethoxyphenyl)-2-(4-(meth)acryloyloxytriethoxyphenyl)propane, 2-(4-(meth)acryloyloxydipropoxyphenyl)-2-(4-(meth)acryloyloxytriethoxyphenyl)propane, 2,2-bis(4-(meth)acryloyloxyisopropoxyphenyl)propane, 2,2-bis(4-(meth)acryloyloxypolyethoxyphenyl)propane (for example, with an average of 2.6 moles of ethoxy group added), 1,4-bis(2-(meth)acryloyloxyethyl)pyromellitate, glycerol di(meth)acrylate, ethylene glycol di(meth)acrylate, diethylene glycol di(meth)acrylate, triethylene glycol di(meth)acrylate, propylene glycol di(meth)acrylate, butylene glycol di(meth)acrylate, neopentyl glycol di(meth)acrylate, polyethylene glycol di(meth)acrylate, 1,3-butanediol di(meth)acrylate, 1,4-butanediol di(meth)acrylate, 1,6-hexanediol di(meth)acrylate, 2-ethyl-1,6-hexanediol di(meth)acrylate, 1,9-nonanediol di(meth)acrylate, 1,10-decanediol di(meth)acrylate, 1,2-bis(3-methacryloyloxy-2-hydroxypropoxy)ethane, and tricyclodecane dimethanol di(meth)acrylate.

Examples of the tri- and higher-functional polymerizable compounds include trimethylolpropane tri(meth)acrylate, trimethylolethane tri(meth)acrylate, trimethylol methane tri(meth)acrylate, pentaerythritol tri(meth)acrylate, pentaerythritol tetra(meth)acrylate, dipentaerythritol penta(meth)acrylate, and 1,7-di(meth)acryloyloxy-2,2,6,6-tetra(meth)acryloyloxymethyl-4-oxaheptane.

The polyfunctional urethanized (meth)acrylic polymerizable compound (A) requires a molecular weight of less than 1,000. In view of strength and impact resistance, the preferred molecular weight is 250 or more and 900 or less, more preferably 300 or more and 800 or less.

The content of the polyfunctional (meth)acrylic polymerizable compound (A) in a stereolithographic resin composition of the present invention is preferably 10 to 80 mass% in total 100 mass% of the polymerizable compounds. In view of even superior strength, toughness, water resistance, and impact resistance in the shaped article when combined with other components, the content of polyfunctional (meth)acrylic polymerizable compound (A) is more preferably 20 to 70 mass%, even more preferably 30 to 60 mass%, most preferably 40 to 50 mass%. The polyfunctional (meth)acrylic polymerizable compound (A) may be used alone, or two or more thereof may be used in combination.

In view of even superior strength, toughness, water resistance, and impact resistance in the shaped article when combined with other components, the content of the polyfunctional (meth)acrylic polymerizable compound (A) in a stereolithographic resin composition of the present invention is preferably 10 to 80 mass%, more preferably 20 to 70 mass%, even more preferably 30 to 60 mass%, most preferably 40 to 50 mass% in total 100 mass% of the stereolithographic resin composition.

[Polyfunctional (Meth)Acrylic Polymerizable Compound (B) with Molecular Weight of 1,000 or More]

In a stereolithographic resin composition of the present invention, the polyfunctional (meth)acrylic polymerizable compound (B) with a molecular weight of 1,000 or more (hereinafter, also referred to simply as "polyfunctional (meth)acrylic polymerizable compound (B)") is used to impart toughness and impact resistance to the shaped article.

The polyfunctional (meth)acrylic polymerizable compound (B) is classified into polyfunctional (meth)acrylic polymerizable compounds that contain an oligomer or polymer structure, and polyfunctional (meth)acrylic polymerizable compounds that do not contain an oligomer or polymer structure. However, in view of superior strength, toughness, and impact resistance in the shaped article when combined with other components, it is preferable to comprise a polyfunctional (meth)acrylic polymerizable compound that contains an oligomer or polymer structure.

The polyfunctional (meth)acrylic polymerizable compound (B) includes polyfunctional urethanized (meth)acrylic polymerizable compound (B)-I containing a urethane bond (hereinafter, also referred to simply as "polyfunctional urethanized (meth)acrylic polymerizable compound (B)-I"), and polyfunctional (meth)acrylic polymerizable compound (B)-II containing no urethane bond. However, in view of superior toughness and impact resistance in the shaped article, it is preferable to comprise a polyfunctional urethanized (meth)acrylic polymerizable compound (B)-I.

The polyfunctional urethanized (meth)acrylic polymerizable compound (B)-I containing an oligomer or polymer structure can be easily synthesized through an addition reaction between a polyol having a polymer backbone (e.g., a polymer structure such as a polyester, a polycarbonate, a polyurethane, a polyether, a polydiene, and a hydrogenated polydiene), a compound having an isocyanate group (-NCO), and a (meth)acrylic compound having a hydroxyl group (-OH). The polyfunctional urethanized (meth)acrylic polymerizable compound (B)-I can also be easily synthesized by allowing lactone or alkylene oxide to undergo a ring-opening addition reaction with a (meth)acrylic compound having a hydroxyl group, and causing the resulting compound with a terminal hydroxyl group to undergo an addition reaction with a compound having an isocyanate group.

The polyfunctional urethanized (meth)acrylic polymerizable compound (B)-I is preferably a (meth)acrylate comprising, within a single molecule, at least one structure selected from the group consisting of a polyester, a polycarbonate, a polyurethane, a polyether, a poly-conjugated diene, and a hydrogenated poly-conjugated diene.

In these structures, examples of the polyester include copolymers of dicarboxylic acids (for example, aromatic dicarboxylic acids such as phthalic acid and isophthalic acid, and unsaturated aliphatic dicarboxylic acids such as maleic acid) and aliphatic diols having 2 to 18 carbon atoms, copolymers of dicarboxylic acids (for example, saturated aliphatic dicarboxylic acids such as adipic acid and sebacic acid) and aliphatic diols having 2 to 18 carbon atoms, β-propiolactone polymers, γ-butyrolactone polymers, δ-valerolactone polymers, ε-caprolactone polymers, and copolymers of these.

Preferred are copolymers of dicarboxylic acids (preferably, aromatic dicarboxylic acids, and unsaturated aliphatic dicarboxylic acids) and aliphatic diols having 2 to 12 carbon atoms, and copolymers of dicarboxylic acids (preferably, saturated aliphatic dicarboxylic acids) and aliphatic glycols having 2 to 12 carbon atoms.

Examples of the polycarbonate include polycarbonates derived from C2 to C18 aliphatic diols, polycarbonates derived from bisphenol A, and polycarbonates derived from C2 to C18 aliphatic diols and bisphenol A. Preferred are polycarbonates derived from C2 to C12 aliphatic diols, polycarbonates derived from bisphenol A, and polycarbonates derived from C2 to C12 aliphatic diols and bisphenol A.

Examples of the polyurethane include polymers of C2 to C18 aliphatic diols and C1 to C18 diisocyanates. Preferred are polymers of C2 to C12 aliphatic diols and C1 to C12 diisocyanates.

Examples of the polyether include polyethylene glycol, polypropylene glycol, polybutylene glycol, and poly(1-methylbutylene glycol).

Examples of the poly-conjugated diene and hydrogenated poly-conjugated diene include 1,4-polybutadiene, 1,2-polybutadiene, polyisoprene, poly(butadiene-isoprene), poly(butadiene-styrene), poly(isoprene-styrene), polyfarnesene, and hydrogenated products of these. **In** view of superior toughness and water resistance, preferred among these are the polyester structures.

**In** view of even superior strength, impact resistance, and water resistance, the polyfunctional urethanized (meth) acrylic polymerizable compound (B)-I is preferably a (meth)acrylate that comprises, within a single molecule, at least one polyol moiety selected from the group consisting of a polyester, a polycarbonate, a polyurethane, a polyether, a poly-conjugated diene, and a hydrogenated poly-conjugated diene each having a structure derived from a C4 to C18 aliphatic chain diol unit having a branched structure.

Examples of the polyester include copolymers with a structure derived from a C4 to C18 aliphatic chain diol unit having a branched structure, and with a structure derived from a C4 to C18 aliphatic chain dicarboxylic acid and/or aromatic dicarboxylic acid unit having no branched structure.

Examples of the polycarbonate include copolymers with a structure derived from a C4 to C18 aliphatic chain diol unit having a branched structure, and with a structure derived from a C4 to C18 aliphatic chain diol unit having no branched structure.

Examples of the polyurethane include structures derived from a C4 to C18 aliphatic chain diol unit having a branched structure, and polycondensates of diisocyanate compounds.

Examples of the polyether include polyethers with a structure derived from a C4 to C18 aliphatic chain diol unit having a branched structure, and polyethers with a structure derived from a C4 to C18 aliphatic chain diol unit having no branched structure.

Examples of the poly-conjugated diene include homopolymers or copolymers of conjugated diene monomers. Examples of the conjugated diene monomers include 1,3-butadiene, isoprene, 2,3-dimethyl-1,3-butadiene, 1,3-pentadiene, and 1,3-hexadiene.

Examples of the hydrogenated poly-conjugated diene include hydrogenated polybutadiene, hydrogenated polyisoprene, and hydrogenated polyisobutylene.

Among these, in view of superior toughness, water resistance, and impact resistance, it is preferable to comprise, as a polymer backbone, at least one structure selected from the group consisting of a polyester, a polycarbonate, a polyether, and a hydrogenated poly-conjugated diene, more preferably at least one structure selected from the group consisting of a polyester and a polycarbonate, even more preferably at least one structure selected from the group consisting of polyesters.

Examples of the diols constituting the C4 to C18 aliphatic chain diol unit having a branched structure include 2-methyl-1,3-propanediol, 2,2-diethyl-1,3-propanediol, 1,3-butanediol, 2-methyl-1,4-butanediol, neopentyl glycol, 3-methyl-1,5-pentanediol, 2-methyl-1,8-octanediol, 2,7-dimethyl-1,8-octanediol, 2-methyl-1,9-nonanediol, 2,8-dimethyl-1,9-nonanediol, 2-methyl-1,10-decanediol, 2,9-dimethyl-1,10-decanediol, 2-methyl-1,11-undecanediol, 2,10-dimethyl-1,11-undecanediol, 2-methyl-1,12-dodecanediol, 2,11-dimethyl-1,12-dodecanediol, 2-methyl-1,13-tridecanediol, 2,12-dimethyl-1,13-tridecanediol, 2-methyl-1,14-tetradecanediol, 2,13-dimethyl-1,14-tetradecanediol, 2-methyl-1,15-pentadecanediol, 2,14-dimethyl-1,15-pentadecanediol, 2-methyl-1,16-hexadecanediol, and 2,15-dimethyl-1,16-hexadecanediol. In view of providing a stereolithographic resin composition having superior curability and low viscosity, preferred for use as polyols are C5 to C12 aliphatic diols having a methyl-group side chain, for example, such as 2-methyl-1,4-butanediol, 3-methyl-1,5-pentanediol, 2-methyl-1,8-octanediol, 2,7-dimethyl-1,8-octanediol, 2-methyl-1,9-nonanediol, and 2,8-dimethyl-1,9-nonanediol, more preferably 2-methyl-1,4-butanediol, 3-methyl-1,5-pentanediol, 2-methyl-1,8-octanediol, and 2,7-dimethyl-1,8-octanediol, even more preferably 3-methyl-1,5-pentanediol, and 2-methyl-1,8-octanediol.

Examples of the compound having an isocyanate group include hexamethylene diisocyanate (HDI), tolylene diisocyanate (TDI), xylylene diisocyanate (XDI), diphenylmethane diisocyanate (MDI), isophorone diisocyanate (IPDI), trimethylhexamethylene diisocyanate (TMHMDI), tricyclodecane diisocyanate (TCDDI), and adamantane diisocyanate (ADI).

Examples of the (meth)acrylic compound having a hydroxyl group include hydroxy (meth)acrylate compounds such as 2-hydroxyethyl (meth)acrylate, 2-hydroxypropyl (meth)acrylate, 2-hydroxybutyl (meth)acrylate, 6-hydroxyhexyl (meth)acrylate, 10-hydroxydecyl (meth)acrylate, 3-chloro-2-hydroxypropyl (meth)acrylate, 2-hydroxy-3-phenoxypropyl (meth)acrylate, glycerin mono(meth)acrylate, 2-hydroxy-3-acryloyloxypropyl (meth)acrylate, 2,2-bis[4-(2-hydroxy-3-(meth)acryloyloxypropoxy)phenyl]propane, 1,2-bis[3-(meth)acryloyloxy-2-hydroxypropoxy]ethane, pentaerythritol tri(meth)acrylate, and tri or tetra(meth)acrylates of dipentaerythritol; and hydroxy(meth)acrylamide compounds such as N-hydroxyethyl(meth)acrylamide, and N,N-bis(2-hydroxyethyl)(meth)acrylamide.

The addition reaction between a compound having an isocyanate group and a (meth)acrylic compound having a hydroxyl group may be performed using known methods and conditions, and is not particularly limited.

Examples of the polyfunctional urethanized (meth)acrylic polymerizable compound (B)-I with no polymer structure include dipentaerythritol penta(meth)acrylate hexamethylene diisocyanate urethane prepolymer.

The polyfunctional urethanized (meth)acrylic polymerizable compound (B)-I requires a molecular weight of 1,000 or more. In view of strength and impact resistance, the preferred molecular weight is 1,500 to 5,000, more preferably 2,000 to 3,000.

Examples of the polyfunctional urethanized (meth)acrylic polymerizable compound (B)-I include bifunctional (meth)acrylic polymerizable compounds, and tri- and higher-functional (meth)acrylic polymerizable compounds.

In view of even superior strength, water resistance, toughness, and impact resistance in the shaped article when combined with other components, the content of the polyfunctional urethanized (meth)acrylic polymerizable compound (B)-I in polyfunctional (meth)acrylic polymerizable compound (B) is preferably 20 mass% or more, more preferably 40 mass% or more, even more preferably 60 mass% or more, most preferably 80 mass% or more in total 100 mass% of the polyfunctional (meth)acrylic polymerizable compound (B). The content of the polyfunctional urethanized (meth)acrylic polymerizable compound (B)-I in polyfunctional (meth)acrylic polymerizable compound (B) may be 100 mass%.

In certain embodiments, in view of even superior strength, water resistance, toughness, and impact resistance in the shaped article when combined with other components, the content of polyfunctional urethanized (meth)acrylic polymerizable compound (B)-I is preferably 1 to 70 mass%, more preferably 5 to 60 mass%, even more preferably 10 to 50 mass%, most preferably 20 to 40 mass% in total 100 mass% of the polymerizable compounds.

The polyfunctional (meth)acrylic polymerizable compound (B)-II with no urethane bond includes bifunctional (meth)acrylic polymerizable compounds, and tri- and higher-functional (meth)acrylic polymerizable compounds.

Examples of the polyfunctional (meth)acrylic polymerizable compound (B)-II with no urethane bond include 2,2-bis(4-(meth)acryloyloxypolyethoxyphenyl)propane (for example, with an average of 25 or more moles of ethoxy group added), and polyethylene glycol di(meth)acrylate.

In view of even superior strength, toughness, water resistance, and impact resistance in the shaped article when combined with other components, the content of the polyfunctional (meth)acrylic polymerizable compound (B) in a stereolithographic resin composition of the present invention is preferably 1 to 80 mass%, more preferably 5 to 70 mass%, even more preferably 10 to 60 mass%, most preferably 20 to 50 mass% in total 100 mass% of the polymerizable compounds. The polyfunctional (meth)acrylic polymerizable compound (B) may be used alone, or two or more thereof may be used in combination.

In view of even superior strength, toughness, water resistance, and impact resistance in the shaped article when combined with other components, the content of the polyfunctional (meth)acrylic polymerizable compound (B) in a stereolithographic resin composition of the present invention is preferably 10 to 60 mass%, more preferably 15 to 65 mass%, even more preferably 20 to 50 mass%, most preferably 25 to 45 mass% in total 100 mass% of the stereolithographic resin composition.

[Monofunctional (Meth)Acrylic Polymerizable Compound (C) That Does Not Comprise Hydroxyl Group and Comprises Cyclic Ether Structure]

In a stereolithographic resin composition of the present invention, the monofunctional (meth)acrylic polymerizable compound (C) that does not comprise a hydroxyl group and comprises a cyclic ether structure (hereinafter, also referred to simply as "monofunctional (meth)acrylic polymerizable compound (C)") is used to reduce viscosity and to impart strength, impact resistance, toughness, and water resistance to the shaped article.

The monofunctional (meth)acrylic polymerizable compound (C) is comprised of a single polymerizable group, a spacer group, and a ring structure having one or more ether groups. In other words, a single polymerizable group is attached to a ring structure having one or more ether groups, via a spacer group. Accordingly, compounds having a structure in which a single polymerizable group is directly attached to a ring structure having one or more ether groups, without the interposition of a spacer group, are excluded from the monofunctional (meth)acrylic polymerizable compound (C) (N-acryloylmorpholine is an example of such compounds).

The polymerizable group of monofunctional (meth)acrylic polymerizable compound (C) is preferably a (meth)acryloxy group.

The spacer group is preferably a linear or branched alkylene group having 1 to 10 carbon atoms.

Examples of the alkylene group include methylene groups, ethylene groups, n-propylene groups, isopropylene groups, trimethylene groups, tetramethylene groups, pentamethylene groups, hexamethylene groups, heptamethylene groups, and octamethylene groups.

The spacer group has preferably 1 to 8 carbon atoms, more preferably 1 to 6 carbon atoms, even more preferably 1 to 4 carbon atoms, particularly preferably 1 to 3 carbon atoms.

The monofunctional (meth)acrylic polymerizable compound (C) does not contain a hydroxyl group because the hydroxyl group, if present, tends to raise the glass transition temperature (Tg) of the homopolymer, leading to poor water resistance.

In relation to the other components, it is not possible to obtain sufficient toughness, water resistance, and impact resistance when the (meth)acrylic polymerizable compound that does not comprise a hydroxyl group and comprises a cyclic ether structure is bifunctional or higher.

The cyclic ether structure of monofunctional (meth)acrylic polymerizable compound (C) is not particularly limited, as long as it is a ring structure with one or more ether groups (-O-). However, the cyclic ether structure is preferably a ring structure with no unsaturated bond, and may be a heteroring structure containing an oxygen atom(s) and a nitrogen atom(s). In view of even superior water resistance, the cyclic ether structure is more preferably a ring structure comprised of oxygen and carbon atoms with no unsaturated bond. Specifically, oxacycloalkane is preferred.

Examples of the oxacycloalkane include ring structures containing one or more oxygen atoms, such as oxacyclopropane (oxirane), oxacyclobutane (oxetane), oxacyclopentane (tetrahydrofuran), oxacyclohexane (tetrahydropyran), dioxacyclohexane (dioxane), and trioxacyclohexane rings. In view of even superior strength, impact resistance, and water resistance in the cured product of a stereolithographic resin composition of the present invention, preferred are ring structures with two or more oxygen atoms, more preferably ring structures with two oxygen atoms.

The ring structure is preferably a five- or higher-membered ring, more preferably a five- to eight-membered ring.

Substituents, for example, such as C1 to C5 alkyl groups, may be introduced into these ring structures. In view of reducing interference with the effect provided by the cyclic ether structure (water resistance in particular), the ring structure is preferably one in which groups such as oxo groups, nitroso groups, and nitro groups are not directly attached.

A certain preferred embodiment is, for example, a stereolithographic resin composition in which the cyclic ether structure of the monofunctional (meth)acrylic polymerizable compound (C) is a ring structure with no oxo group. For example, when dioxolans and dioxanes have oxo groups, they may form carbonate groups (-O-(C=O)-O-), which results in an excessive increase in polarity, leading to a serious decrease in water resistance.

The ring structure should have one or more heteroatoms, and may contain two or more heteroatoms. However, in view of lowering the boiling point of the compound and helping reduce odor generation, as well as achieving superior impact resistance, the number of heteroatoms is preferably two.

In view of even superior strength and impact resistance in the cured product of a stereolithographic resin composition of the present invention when combined with other components, preferred are dioxolans (such as 1,2-dioxolan, and 1,3-dioxolan), oxacyclopentane (tetrahydrofuran), oxacyclohexane (tetrahydropyran), and dioxacyclohexane (dioxane), more preferably dioxanes, even more preferably dioxolans and dioxanes. In view of notably superior impact resistance, dioxanes are particularly preferred.

Concerning these rings, the positions of oxygen atoms are not particularly limited, and the dioxane may be any of 1,2-dioxane, 1,3-dioxane, and 1,4-dioxane.

A homopolymer of monofunctional (meth)acrylic polymerizable compound (C) has a glass transition temperature (Tg) of preferably 50°C or less, more preferably 45°C or less, even more preferably 40°C or less. A homopolymer of monofunctional (meth)acrylic polymerizable compound (C) has a glass transition temperature (Tg) of preferably -20°C or more, more preferably -15°C or more, even more preferably 5°C or more.

When a homopolymer of monofunctional (meth)acrylic polymerizable compound (C) has a glass transition temperature (Tg) close to the temperature under actual use of the shaped article, for example, such as in intraoral applications (preferably, dental material applications), the impact resistance in particular tends to greatly improve when the glass transition temperature (Tg) corresponds to a temperature range extending from around room temperature to body temperature, specifically from 15°C to 40°C.

The glass transition temperature (Tg) of the homopolymer can be measured following traditionally known methods, using, for example, a viscoelasticity meter (rheometer) or differential scanning calorimeter (DSC).

Specifically, the glass transition temperature Tg of the homopolymer can be determined by measuring the dynamic viscoelasticity of the homopolymer of monofunctional (meth)acrylic polymerizable compound (C) using a rotational rheometer (AR2000 manufactured by TA Instruments Japan Inc.). In this dynamic viscoelasticity measurement, the measurement can be performed under the set conditions of 10 Hz frequency, 10 N load, 0.1% displacement, and 20 µNm torque, and the temperature at which tan δ reaches its peak can be determined as the glass transition temperature Tg.

Examples of the monofunctional (meth)acrylic polymerizable compound (C) comprising a cyclic ether structure include mono(meth)acrylates such as:

epoxy group-containing (meth)acrylates such as glycidyl (meth)acrylate, 2-epoxyethyl (meth)acrylate, 3-epoxypropyl (meth)acrylate, 4-epoxybutyl (meth)acrylate, 5-epoxypentyl (meth)acrylate, 3,4-epoxycyclohexylmethyl (meth)acrylate, and 3,4-epoxycyclohexylethyl (meth)acrylate;

furan ring-containing (meth)acrylates such as tetrahydrofurfuryl (meth)acrylate;

oxetane ring-containing (meth)acrylates such as (3-ethyloxetan-3-yl)methyl (meth)acrylate;

dioxolan ring-containing (meth)acrylates such as (2-methyl-2-ethyl-1,3-dioxolan-4-yl)methyl (meth)acrylate, (2-methyl-1,3-dioxolan-4-yl)methyl (meth)acrylate, (2-ethyl-1,3-dioxolan-4-yl)methyl (meth)acrylate, 2-((2-methyl-1,3-dioxolan-4-yl)methoxy)ethyl (meth)acrylate, and 2-((2-ethyl-1,3-dioxolan-4-yl)methoxy)ethyl (meth)acrylate;

dioxane ring-containing (meth)acrylates such as cyclic trimethylolpropane formal (meth)acrylate (another name: (5-ethyl-1,3-dioxan-5-yl)methyl (meth)acrylate); and

tetrahydropyran ring-containing (meth)acrylates such as 2-[(2-tetrahydropyranyl)oxy]ethyl (meth)acrylate.

In view of superior strength and impact resistance in the cured product of a stereolithographic resin composition of the present invention, preferred among these are tetrahydrofurfuryl (meth)acrylate, (2-methyl-2-ethyl-1,3-dioxolan-4-yl)methyl (meth)acrylate, (2-methyl-1,3-dioxolan-4-yl)methyl (meth)acrylate, (2-ethyl-1,3-dioxolan-4-yl)methyl (meth)acrylate, 2-((2-methyl-1,3-dioxolan-4-yl)methoxy)ethyl (meth)acrylate, 2-((2-ethyl-1,3-dioxolan-4-yl)methoxy)ethyl (meth)acrylate, cyclic trimethylolpropane formal (meth)acrylate, and 2-[(2-tetrahydropyranyl)oxy]ethyl (meth)acrylate, more preferably (2-methyl-2-ethyl-1,3-dioxolan-4-yl)methyl (meth)acrylate, (2-methyl-1,3-dioxolan-4-yl)methyl (meth)acrylate, (2-ethyl-1,3-dioxolan-4-yl)methyl (meth)acrylate, 2-((2-methyl-1,3-dioxolan-4-yl)methoxy)ethyl (meth)acrylate, 2-((2-ethyl-1,3-dioxolan-4-yl)methoxy)ethyl (meth)acrylate, and cyclic trimethylolpropane formal (meth)acrylate. In view of notably superior impact resistance, cyclic trimethylolpropane formal (meth)acrylate is even more preferred.

The content of the monofunctional (meth)acrylic polymerizable compound (C) in a stereolithographic resin composition of the present invention is preferably 1.0 to 60 mass% in total 100 mass% of the polymerizable compounds. In view of achieving low viscosity in the stereolithographic resin composition and providing excellent strength and impact resistance in the shaped article without impairing strength, toughness, and water resistance when combined with other components, the content of monofunctional (meth)acrylic polymerizable compound (C) is more preferably 5.0 to 50 mass%, even more preferably 10 to 40 mass%, most preferably 15 to 30 mass%.

The monofunctional (meth)acrylic polymerizable compound (C) may be used alone, or two or more thereof may be used in combination.

In certain embodiments, in view of even superior strength, water resistance, and impact resistance in the shaped article when combined with other components, the content of monofunctional (meth)acrylic polymerizable compound (C) is preferably 1.0 to 60 mass%, more preferably 5.0 to 50 mass%, even more preferably 10 to 40 mass%, most preferably 15 to 30 mass% in total 100 mass% of the stereolithographic resin composition.

In another preferred embodiment, the mass ratio of the content of monofunctional (meth)acrylic polymerizable compound (C) and that of polyfunctional (meth)acrylic polymerizable compound (A) is preferably (A)/(C) > 1. With the content of polyfunctional (meth)acrylic polymerizable compound (A) exceeding that of monofunctional (meth)acrylic polymerizable compound (C), it is possible to achieve even superior strength, water resistance, and impact resistance in the shaped article when combined with other components.

In yet another preferred embodiment, the mass ratio of the content of monofunctional (meth)acrylic polymerizable compound (C) and that of polyfunctional (meth)acrylic polymerizable compound (B) is preferably (B)/(C) > 1. With the content of polyfunctional (meth)acrylic polymerizable compound (B) exceeding that of monofunctional (meth)acrylic polymerizable compound (C), it is possible to achieve even superior strength, water resistance, and impact resistance in the shaped article when combined with other components.

In still another preferred embodiment, the mass ratio of the content of monofunctional (meth)acrylic polymerizable compound (C) and the total content of polyfunctional (meth)acrylic polymerizable compound (A) and polyfunctional (meth)acrylic polymerizable compound (B) is preferably ((A)+(B))/(C) > 1. With the total content of polyfunctional (meth)acrylic polymerizable compound (A) and polyfunctional (meth)acrylic polymerizable compound (B) exceeding the content of monofunctional (meth)acrylic polymerizable compound (C), it is possible to achieve even superior strength, toughness, water resistance, and impact resistance in the shaped article.

The mass ratio ((A)+(B))/(C) is more preferably 1.5 or more, even more preferably 2 or more, most preferably 3 or more.

The mass ratio ((A)+(B))/(C) is preferably 20 or less, more preferably 15 or less, even more preferably 10 or less, most preferably 6 or less.

A certain preferred embodiment is, for example, a stereolithographic resin composition that comprises a polyfunctional (meth)acrylic polymerizable compound (A) with a molecular weight of less than 1,000, a polyfunctional (meth)acrylic polymerizable compound (B) with a molecular weight of 1,000 or more, a monofunctional (meth)acrylic polymerizable compound (C) that does not comprise a hydroxyl group and comprises a cyclic ether structure, and a photopolymerization initiator (D),

wherein the polyfunctional (meth)acrylic polymerizable compound (A) comprises a polyfunctional urethanized (meth) acrylic polymerizable compound (A)-I containing a urethane bond,
the polyfunctional urethanized (meth)acrylic polymerizable compound (A)-I is a (meth)acrylate that does not contain a polymer structure within a single molecule,
the polyfunctional (meth)acrylic polymerizable compound (B) comprises a polyfunctional urethanized (meth)acrylic polymerizable compound (B)-I containing a urethane bond,
the polyfunctional urethanized (meth)acrylic polymerizable compound (B)-I is a (meth)acrylate that comprises, within a single molecule, at least one structure selected from the group consisting of a polyester, a polycarbonate, a polyurethane, a polyether, a poly-conjugated diene, and a hydrogenated poly-conjugated diene, and
the cyclic ether structure of the monofunctional (meth)acrylic polymerizable compound (C) is a dioxane.

In the above preferred embodiment, the monofunctional (meth)acrylic polymerizable compound (C) is preferably a mono(meth)acrylate that contains a dioxane as the cyclic ether structure.

In the above preferred embodiment, the polyfunctional urethanized (meth)acrylic polymerizable compound (B)-I is preferably a (meth)acrylate that comprises, within a single molecule, at least one polyol moiety selected from the group consisting of a polyester, a polycarbonate, a polyurethane, a polyether, a poly-conjugated diene, and a hydrogenated poly-conjugated diene each having a structure derived from a C4 to C18 aliphatic chain diol unit having a branched structure.

In any of the foregoing preferred embodiments, it is preferable that the stereolithographic resin composition be essentially free of a (meth)acrylamide oligomer (for example, with a molecular weight of 1,000 or less, or with a molecular weight of 1,000 or more).

Here, by "essentially free of a (meth)acrylamide oligomer", it means that the content of (meth)acrylamide oligomers is less than 5 mass%, preferably less than 1 mass%, more preferably less than 0.1 mass%, even more preferably less than 0.01 mass% in total 100 mass% of the stereolithographic resin composition.

In any of the foregoing preferred embodiments, the type and content of each component may be modified as appropriate based on the descriptions of this specification.

[Photopolymerization Initiator (D)]

The photopolymerization initiator (D) used in the present invention may be selected from polymerization initiators used in industry, preferably from photopolymerization initiators used in dentistry.

Examples of the photopolymerization initiator (D) include (bis)acylphosphine oxides, thioxanthones or quaternary ammonium salts of thioxanthones, ketals, α-diketones, coumarins, anthraquinones, benzoin alkyl ether compounds, and α-aminoketone compounds. The photopolymerization initiator (D) may be used alone, or two or more thereof may be used in combination.

Preferably, the photopolymerization initiator (D) is at least one selected from the group consisting of (bis) acylphosphine oxides and α-diketones. In this way, a stereolithographic resin composition can be obtained that has excellent photocurability both in the ultraviolet and visible regions, and that shows sufficient photocurability even when the light source is a laser, a halogen lamp, a light emitting diode (LED), or a xenon lamp.

Examples of acylphosphine oxides in the (bis)acylphosphine oxides include 2,4,6-trimethylbenzoyldiphenylphosphine oxide, 2,6-dimethoxybenzoyldiphenylphosphine oxide, 2,6-dichlorobenzoyldiphenylphosphine oxide, 2,4,6-trimethylbenzoylmethoxyphenylphosphine oxide, 2,4,6-trimethylbenzoylethoxyphenylphosphine oxide, 2,3,5,6-tetramethylbenzoyldiphenylphosphine oxide, benzoyl di(2,6-dimethylphenyl)phosphonate, sodium salts of 2,4,6-trimethylbenzoylphenylphosphine oxide, potassium salts of 2,4,6-trimethylbenzoylphenylphosphine oxide, and ammonium salts of 2,4,6-trimethylbenzoyldiphenylphosphine oxide.

Examples of bisacylphosphine oxides include bis(2,6-dichlorobenzoyl)phenylphosphine oxide, bis(2,6-dichlorobenzoyl)-2,5-dimethylphenylphosphine oxide, bis(2,6-dichlorobenzoyl)-4-propylphenylphosphine oxide, bis(2,6-dichlorobenzoyl)-1-naphthylphosphine oxide, bis(2,6-dimethoxybenzoyl)phenylphosphine oxide, bis(2,6-dimethoxybenzoyl)-2,4,4-trimethylpentylphosphine oxide, bis(2,6-dimethoxybenzoyl)-2,5-dimethylphenylphosphine oxide, bis(2,4,6-trimethylbenzoyl)phenylphosphine oxide, and bis(2,5,6-trimethylbenzoyl)-2,4,4-trimethylpentylphosphine oxide. Other examples include compounds mentioned in JP 2000-159621 A.

Among these (bis)acylphosphine oxides, particularly preferred as photopolymerization initiator (D) are 2,4,6-trimethylbenzoyldiphenylphosphine oxide, 2,4,6-trimethylbenzoylmethoxyphenylphosphine oxide, bis(2,4,6-trimethylbenzoyl)phenylphosphine oxide, and sodium salts of 2,4,6-trimethylbenzoylphenylphosphine oxide.

Examples of the α-diketones include diacetyl, benzyl, camphorquinone, 2,3-pentadione, 2,3-octadione, 9,10-phenanthrenequinone, 4,4'-oxybenzyl, and acenaphthenequinone. Camphorquinone is particularly preferred when the light source used is a visible light source.

The content of the photopolymerization initiator (D) in a stereolithographic resin composition of the present invention is not particularly limited. However, in view of curability and other properties of the stereolithographic resin composition obtained, the content of photopolymerization initiator (D) is preferably 0.01 to 20 parts by mass relative to total 100 parts by mass of the polymerizable compounds. When the content of photopolymerization initiator (D) is less than 0.01 parts by mass, polymerization may not sufficiently proceed to form a shaped product. The content of photopolymerization initiator (D) is more preferably 0.05 parts or more by mass, even more preferably 0.1 parts or more by mass, particularly preferably 0.5 parts or more by mass relative to total 100 parts by mass of the polymerizable compounds. When the content of photopolymerization initiator (D) is more than 20 parts by mass, the photopolymerization initiator (D) may precipitate out of the stereolithographic resin composition when the solubility of the photopolymerization initiator itself is low. The content of photopolymerization initiator (D) is more preferably 15 parts or less by mass, even more preferably 10 parts or less by mass, particularly preferably 5.0 parts or less by mass relative to total 100 parts by mass of the polymerizable compounds.

A stereolithographic resin composition of the present invention is not particularly limited, and may comprise other components for example, as long as it comprises the polyfunctional (meth)acrylic polymerizable compound (A) with a molecular weight of less than 1,000, the polyfunctional (meth)acrylic polymerizable compound (B) with a molecular weight of 1,000 or more, the monofunctional (meth)acrylic polymerizable compound (C) that comprises a cyclic ether structure, and the photopolymerization initiator (D). The method of production of a stereolithographic resin composition of the present invention is not particularly limited, and a stereolithographic resin composition of the present invention can be produced following known methods of producing stereolithographic resin compositions.

A stereolithographic resin composition of the present invention may comprise a polymerization accelerator to improve photocurability, provided that it does not hinder the intent and purpose of the present invention. Examples of the polymerization accelerator include ethyl 4-(N,N-dimethylamino)benzoate, methyl 4-(N,N-dimethylamino)benzoate, n-butoxyethyl 4-(N,N-dimethylamino)benzoate, 2-(methacryloyloxy)ethyl 4-(N,N-dimethylamino)benzoate, 4-(N,N-dimethylamino)benzophenone, and butyl 4-(N,N-dimethylamino)benzoate. In view of imparting superior curability to the stereolithographic resin composition, preferred is at least one selected from the group consisting of ethyl 4-(N,N-dimethylamino)benzoate, n-butoxyethyl 4-(N,N-dimethylamino)benzoate, and 4-(N,N-dimethylamino)benzophenone.

A stereolithographic resin composition of the present invention may further comprise a filler mixed therein to adjust paste properties or to improve the mechanical strength of the shaped article of the stereolithographic resin composition. Examples of the filler include organic fillers, inorganic fillers, and organic-inorganic composite fillers. The filler may be used alone, or two or more thereof may be used in combination.

Examples of organic filler materials include polymethyl methacrylate, polyethyl methacrylate, a methyl methacrylate-ethyl methacrylate copolymer, crosslinked polymethyl methacrylate, crosslinked polyethyl methacrylate, polyesters, polyamides, polycarbonates, polyphenylene ethers, polyoxymethylene, polyvinyl chloride, polystyrene, polyethylene, polypropylene, chloroprene rubber, nitrile rubber, an ethylene-vinyl acetate copolymer, a styrene-butadiene copolymer, an acrylonitrile-styrene copolymer, and an acrylonitrile-styrene-butadiene copolymer. These may be used alone, or two or more thereof may be used in combination. The shape of organic filler is not particularly limited, and the particle diameter of filler may be appropriately selected for use.

Examples of inorganic filler materials include quartz, silica, alumina, silica-titania, silica-titania-barium oxide, silica-zirconia, silica-alumina, lanthanum glass, borosilicate glass, soda glass, barium glass, strontium glass, glass-ceramic, aluminosilicate glass, barium boroaluminosilicate glass, strontium boroaluminosilicate glass, fluoroaluminosilicate glass, calcium fluoroaluminosilicate glass, strontium fluoroaluminosilicate glass, barium fluoroaluminosilicate glass, and strontium calcium fluoroaluminosilicate glass. These may be used alone, or two or more thereof may be used in combination. The shape of inorganic filler is not particularly limited, and the particle diameter of filler may be appropriately selected for use, such as irregularly shaped fillers, and spherical fillers.

A stereolithographic resin composition of the present invention may comprise a polymer to alter properties such as flexibility and flowability, provided that it does not hinder the intent and purpose of the present invention. Examples include natural rubber, synthetic polyisoprene rubber, liquid polyisoprene rubber and hydrogenated products thereof, polybutadiene rubber, liquid polybutadiene rubber and hydrogenated products thereof, styrene-butadiene rubber, chloroprene rubber, ethylene-propylene rubber, acryl rubber, isoprene-isobutylene rubber, acrylonitrile-butadiene rubber, and styrene elastomers. Specific examples of other polymers that may be added include a polystyrene-polyisoprene-polystyrene block copolymer, a polystyrene-polybutadiene-polystyrene block copolymer, a poly(α-methylstyrene)-polybutadiene-poly(α-methylstyrene) block copolymer, a poly(p-methylstyrene)-polybutadiene-poly(p-methylstyrene) block copolymer, and hydrogenated products of these.

A stereolithographic resin composition of the present invention may optionally comprise a softener. Examples of the softener include petroleum-based softeners such as paraffinic, naphthenic, and aromatic process oils, and vegetable oil-base softeners such as paraffin, peanut oil, and rosin. These softeners may be used alone, or two or more thereof may be used in combination. The softener content is not particularly limited, provided that it does not hinder the intent and purpose of the present invention. Typically, the softener content is 200 parts or less by mass, preferably 100 parts or less by mass relative to total 100 parts by mass of the polymerizable compounds.

] A stereolithographic resin composition of the present invention may comprise a known stabilizer, in order to inhibit deterioration or adjust photocurability. Examples of such stabilizers include polymerization inhibitors, ultraviolet absorbers, and antioxidants.

] Examples of the polymerization inhibitors include hydroquinone, hydroquinone monomethyl ether, dibutylhydroquinone, dibutylhydroquinone monomethyl ether, 4-t-butyl catechol, 2-t-butyl-4,6-dimethylphenol, 2,6-di-t-butylphenol, and 3,5-di-t-butyl-4-hydroxytoluene. The content of the polymerization inhibitors is preferably 0.001 to 5.0 parts by mass relative to total 100 parts by mass of the polymerizable compounds.

A stereolithographic resin composition of the present invention may comprise a known additive, in order to adjust shades or paste properties. Examples of such additives include pigments, dyes, organic solvents, and thickeners.

A stereolithographic resin composition of the present invention exhibits excellent strength, toughness, water resistance, and impact resistance in the shaped article, in addition to excellent fabricability. This makes a stereolithographic resin composition of the present invention usable in applications where such advantages can be exploited, including intraoral applications. Examples of intraoral applications include dental materials such as denture base materials, and dental occlusal splints; and materials for treating sleep disorder (particularly, appliances for the treatment of sleep apnea). A stereolithographic resin composition of the present invention is particularly suited for dental occlusal splints, denture base materials, and appliances for treating sleep apnea.

The shaped article of a stereolithographic resin composition of the present invention may have a shape that depends on intended use. In a stereolithographic resin composition of the present invention, the type and content of polyfunctional (meth)acrylic polymerizable compound (A), polyfunctional (meth)acrylic polymerizable compound (B), monofunctional (meth)acrylic polymerizable compound (C), and photopolymerization initiator (D), as well as optional components (such as polymerization accelerators, fillers, polymers, softeners, stabilizers, and additives) may be optionally adjusted for different uses, for example, dental occlusal splints, denture base materials, and appliances for treating sleep apnea.

A stereolithographic resin composition of the present invention can be used in a wide variety of applications by taking advantage of its properties, specifically, the superior shape precision due to the low rate of volume shrinkage upon curing with light, and the ability to produce shaped products, three-dimensional shaped articles, or other shaped articles having superior strength, toughness, water resistance, and impact resistance. For example, a stereolithographic resin composition of the present invention can be used for stereolithographic production of three-dimensional shaped articles, and production of various shaped products, for example, film-shaped articles or molded articles produced by a technique such as film casting or casting, and molds for coating and vacuum molding applications.

A stereolithographic resin composition of the present invention is particularly suited for stereolithography such as above. In stereolithography applications, a stereolithographic resin composition of the present invention enables smooth production of a three-dimensional shaped article having superior toughness and mechanical characteristics while ensuring superior dimensional accuracy with a maintained low rate of volume shrinkage at the time of curing with light.

A stereolithographic resin composition of the present invention exhibits excellent strength, toughness, water resistance, and impact resistance, and possesses adequate flexibility with mechanical characteristics that are not excessive high. This makes a stereolithographic resin composition of the present invention suitable in intraoral applications, particularly as orthodontic mouthpieces or aligners used to adjust tooth alignment and/or jaw position, or dental soft splints such as mouthguards used in sports.

Another embodiment of the present invention is a method for producing a three-dimensional shaped article by a stereolithography method using any of the stereolithographic resin compositions described above. The stereolithography method is not particularly limited, and may employ vat photopolymerization techniques such as laser SLA (Stereolithography Apparatus), and DLP (digital light processing) SLA. LFS (Low Force Stereolithography) represents another applicable SLA technique.

In stereolithography using a stereolithographic resin composition of the present invention, any known stereolithography method and device (for example, a stereolithography device such as the DIGITALWAX® 028J-Plus manufactured by DWS) may be used. In the present invention, the light energy used to cure the resin is preferably an active energy beam. As used herein, "active energy beam" means an energy ray capable of curing a stereolithographic resin composition, and includes, for example, ultraviolet light, an electron beam, X-rays, radiant rays, and high-frequency waves. For example, the active energy beam may be ultraviolet light of 300 to 400 nm wavelengths. The light source of active energy beam may be, for example, a laser such as an Ar laser or a He-Cd laser; or a lighting such as a halogen lamp, a xenon lamp, a metal halide lamp, an LED, a mercury lamp, or a fluorescent lamp. Lasers are particularly preferred. When the light source is a laser, the fabrication time can be reduced by increasing the energy level, and a three-dimensional shaped article of high shape precision can be obtained by taking advantage of the desirable convergence of a laser beam.

Stereolithography using a stereolithographic resin composition of the present invention may use any known method and any known stereolithography system, and the method and device are not particularly limited, as noted above.

However, a typical example of a stereolithography method preferred for use in the present invention is a method that produces the desired final three-dimensional shaped article through a repeated procedure that includes a step of forming a cured layer by selectively applying an active energy beam to the stereolithographic resin composition so as to obtain a cured layer having a desired pattern, and a step of continuously forming another cured layer on the previously formed cured layer by similarly applying an active energy beam to a newly supplied, uncured liquid of the stereolithographic resin composition. The resulting three-dimensional shaped article may be used as is, or, depending on the application, it may be used after improving mechanical characteristics, shape stability, or other properties by, for example, post-curing the product under applied light or heat.

The three-dimensional shaped article obtained by stereolithography is not limited to a particular structure, shape, or size, and these may be decided according to use. Typical examples of areas to which the stereolithography method of the present invention is applicable include production of various models and molds, including, for example, models for assessing external designs in a designing process; models for checking functions of components or parts; resin molds for making casting molds; base models for making molds; and direct molds for prototype molds. More specifically, the stereolithography method of the present invention is applicable to, for example, production of models or work models for precision components and parts, electrical and electronic components, furniture, architectural structures, automobile parts, various containers and vessels, castings, molds, and masters. By taking advantage of the excellent strength and toughness of the shaped article of the stereolithographic resin composition, the stereolithography method of the present invention can be very effectively used for, for example, complex-shape cushioning materials in structures (for example, architectural structures), and molds for vacuum molding.

EXAMPLES

The following describes the present invention in greater detail by way of Examples. It should be noted, however, that the present invention is in no way limited by the following Examples, and various changes may be made by a person with ordinary skill in the art within the technical idea of the present invention.

The components used for the polymerizable compositions according to Examples and Comparative Examples are described below, along with the abbreviations.

[Polyfunctional Urethanized (Meth)Acrylic Polymerizable Compound (A)-I]

UDMA: 2,2,4-trimethylhexamethylenebis(2-carbamoyloxyethyl)dimethacrylate (manufactured by Kyoeisha Chemical Co., Ltd.; molecular weight: 471)

AH-600: phenyl glycidyl ether acrylate hexamethylene diisocyanate urethane prepolymer (manufactured by Kyoeisha Chemical Co., Ltd.; molecular weight: 612)

U4TH:   N,N'-(2,2,4-trimethylhexamethylene)bis[2-(aminocarboxy)propane-1,3-diol]tetramethacrylate (manufactured by Kyoeisha Chemical Co., Ltd.; molecular weight: 673)

[Polyfunctional (Meth)Acrylic Polymerizable Compound (A)-II Containing no Urethane Bond]

D2.6E: 2,2-bis(4-methacryloyloxypolyethoxyphenyl)propane (with an average of 2.6 moles of ethoxy group added; BPE-100 manufactured by Shin-Nakamura Chemical Co., Ltd.; molecular weight: approximately 478)

[Polyfunctional Urethanized (Meth)Acrylic Polymerizable Compound (B)-I]

<Synthesis Example 1> [Production of Polyfunctional Urethanized (Meth)Acrylic Polymerizable Compound (B)-I-1]

(1) A 5 L four-neck flask equipped with a stirrer, a thermostat, a thermometer, and a condenser was charged with 250 g of isophorone diisocyanate and 0.15 g of di-n-butyltin dilaurate, and the mixture was heated to 70°C while being stirred.

(2) Separately, 2,500 g of polyester polyol (Kuraray Polyol® P-2050 manufactured by Kuraray Co., Ltd.; a polyol composed of sebacic acid and 3-methyl-1,5-pentanediol; weight-average molecular weight Mw: 2,000) was added into a dropping funnel equipped with a side tube, and the solution in the dropping funnel was dropped into the flask of (1). Here, the solution was dropped at a constant rate over a time period of 4 hours with the temperature inside the flask held at 65 to 75°C while stirring the solution in the flask of (1). After dropping, the mixture was stirred at the same temperature for 2 hours to allow reaction.

(3) Thereafter, a homogenous solution prepared by adding 150 g of 2-hydroxyethyl acrylate and 0.4 g of hydroquinone monomethyl ether into a different dropping funnel was dropped into the flask of (2) at a constant rate over a time period of 2 hours with the temperature inside the flask held at 55 to 65°C, and a reaction was allowed for 4 hours at the maintained solution temperature of 70 to 80°C in the flask to obtain a urethanized (meth)acrylic polymerizable compound (B)-I-1. The polyfunctional urethanized (meth)acrylic polymerizable compound (B)-I-1 had a weight-average molecular weight Mw of 2,600 as measured by GPC analysis.

The weight-average molecular weight Mw of the compound synthesized above means a weight-average molecular weight in terms of polystyrene equivalents, as determined by gel permeation chromatography (GPC).

Polyfunctional urethanized (meth)acrylic polymerizable compound (B)-I-2: polyether-based urethane acrylate; molecular weight: 1,600; NK Oligo UA-160TM manufactured by Shin-Nakamura Chemical Co., Ltd.

[Polyfunctional (Meth)Acrylic Polymerizable Compound (B)-II Containing no Urethane Bond]

A-BPE-30: 2,2-bis(4-methacryloyloxypolyethoxyphenyl)propane (with an average of 30 moles of ethoxy group added; A-BPE-30 manufactured by Shin-Nakamura Chemical Co., Ltd.; molecular weight: approximately 1,656)

[Monofunctional (Meth)Acrylic Polymerizable Compound (C)]

CTMPFA: cyclic trimethylol propane formal acrylate (manufactured by SARTOMER; Tg of homopolymer: 32°C)

MEDOMA: (2-methyl-2-ethyl-1,3-dioxolan-4-yl)methyl acrylate (MEDOL-10 manufactured by Osaka Organic Chemical Industry Ltd.; Tg of homopolymer: -7°C)

[Photopolymerization Initiator (D)]

TPO: 2,4,6-trimethylbenzoyldiphenylphosphine oxide

[Polymerization Inhibitor]

BHT: 3,5-di-t-butyl-4-hydroxytoluene

[Examples 1 to 9 and Comparative Examples 1 to 5]

The components were mixed at an ordinary temperature (20°C $\pm$ 15°C; JIS (Japanese Industrial Standards) Z 8703:1983) in the amounts (parts by mass) shown in Tables 1 and 2 to prepare pastes representing stereolithographic resin compositions according to Examples 1 to 9 and Comparative Examples 1 and 5.

The stereolithographic resin composition was fabricated into a specimen with the dimensions (64.0 mm in length, 10.0 mm in width, and 3.3 mm in thickness) described in JDMAS 253:2020 (3D printing photopolymerizable resin for dental hard splints) 6.4, using a stereolithography device (DIGITALWAX® 020D manufactured by DWS). Various tests were conducted using this specimen as a cured product of the stereolithographic resin composition.

<Strength (Flexural Modulus, Flexural Strength), Toughness (Displacement of Flexural Fracture Point)>

The cured products of the stereolithographic resin compositions according to Examples and Comparative Examples were stored in air for 1 day, and measured for strength (flexural modulus, flexural strength) and toughness (displacement of flexural fracture point). In the measurement, a flexure test was conducted in compliance with JDMAS 253:2020 (3D printing photopolymerizable resin for dental hard splints) 6.4 at a span length of 50 mm and a crosshead speed of 5 mm/min, using a universal testing machine (Autograph AG-I 100kN manufactured by Shimadzu Corporation) (n = 5). The mean values of the measurements are presented in Tables 1 and 2.

In view of applicability particularly suited as dental soft splints, the preferred flexural modulus range is 300 MPa or more and less than 1,500 MPa, more preferably 400 MPa or more and 1,000 MPa or less, even more preferably 500 MPa or more and 750 MPa or less.

The preferred flexural strength (three-point flexural strength) is 10 MPa or more, more preferably 15 MPa or more, even more preferably 20 MPa or more.

In view of applicability particularly suited as dental soft splints, the preferred flexural strength is less than 50 MPa.

As for the displacement of fracture point, it is preferable to have no fracture.

The displacement of fracture point was evaluated based on the following criteria, with ratings "A" (favorable toughness) and "B" (moderate toughness) considered acceptable.

<Evaluation Criteria>

A: The cured product fractured at a displacement of 15 mm or more.
B: The cured product fractured at a displacement of more than 10 mm and less than 15 mm.
C: The cured product fractured at a displacement of 10 mm or less.

<Water Resistance>

The cured products of the stereolithographic resin compositions according to Examples and Comparative Examples were immersed in 37°C water for 168 hours, and measured for flexural strength in the same manner as in the flexural strength test above (n = 5). The mean values of the measurements are presented in Tables 1 and 2. Assuming that the measured flexural strength in the toughness evaluation above is the initial flexural strength, a rate of change (percentage decrease) in flexural strength of 20% or less indicates excellent water resistance, as indicated below. In Tables 1 and 2, the flexural strength after immersion in 37°C water for 168 hours is denoted as "Flexural strength after immersion."

Rate of change (percentage decrease) in flexural strength (%) = [{initial flexural strength (MPa) - flexural strength after immersion in 37°C water for 168 hours (MPa)} / initial flexural strength (MPa)] $\times$ 100

<Impact Resistance>

The cured product of the stereolithographic resin composition from each Example and Comparative Example was placed on the two specimen supports of a flexure test jig. A single SUS304 stainless steel ball with a diameter Ø of 5/8 inches (a stainless steel ball available as Model SUS-5/8 from the ASONE Comprehensive Laboratory Equipment & Supplies Catalog) was then dropped freely from a height of either 80 cm or 60 cm, striking the center of the cured product. Preferably, the cured product should not fracture in the test.
The impact resistance was evaluated based on the following criteria, with ratings "A" (notably favorable impact resistance) and "B" (favorable impact resistance) considered acceptable.

<Evaluation Criteria>

A: The cured product did not break after dropping the steel ball from a height of 80 cm.
B: The cured product did not break after dropping the steel ball from a height of 60 cm but broke when the steel ball was dropped from 80 cm.
C: The cured product broke after dropping the steel ball from a height of 60 cm.

[Table 1]

| Components (parts by mass) | | | Ex. 1 | Ex. 2 | Ex. 3 | Ex. 4 | Ex. 5 | Ex. 6 | Ex. 7 | Ex. 8 | Ex. 9 |
|---|---|---|---|---|---|---|---|---|---|---|---|
| Raw materials | (A) | UDMA | 45 | 35 | 55 | 45 | | 40 | | 45 | 45 |
| | | AH-600 | | | | | 45 | | | | |
| | | U4TH | | | | | | 5 | | | |
| | | D2.6E | | | | | | | 45 | | |
| | (B) | Polyfunctional urethanized (meth)acrylic polymerizable compound (B)-I-1 | 35 | 40 | 30 | 35 | 35 | 35 | 35 | | |
| | | Polyfunctional urethanized (meth)acrylic polymerizable compound (B)-I-2 | | | | | | | | 35 | |
| | | A-BPE-30 | | | | | | | | | 35 |
| | (C) | CTMPFA | 20 | 25 | 15 | | 20 | 20 | 20 | 20 | 20 |
| | | MEDOMA | | | | 20 | | | | | |
| | (D) | TPO | 2.0 | 2.0 | 2.0 | 2.0 | 2.0 | 1.5 | 3.0 | 2.0 | 3.0 |
| | | BHT | 0.5 | 0.5 | 0.5 | 0.5 | 0.5 | 0.5 | 0.5 | 0.5 | 0.5 |
| Properties | Strength | Flexural strength | (MPa) | 25 | 21 | 30 | 22 | 23 | 28 | 28 | 23 | 19 |
| | | Flexural modulus | (MPa) | 650 | 520 | 720 | 580 | 620 | 700 | 920 | 550 | 480 |
| | Toughness | Displacement of fracture point | | A | A B | B | A | A | B | B | A | B |
| | Water resistance | Flexural strength after immersion | (MPa) | 23 | 18 | 28 | 18 | 20 | 26 | 27 | 19 | 16 |
| | | Percentage decrease | (%) | 8.0 | 14 | 6.7 | 18 | 13 | 7.1 | 3.5 | 17 | 16 |
| | Impact resistance | Steel ball drop test | | A | A | A | B | A | A | B | B | A |

[Table 2]

| Components (parts by mass) | | | | Com. Ex. 1 | Com. Ex. 2 | Com. Ex. 3 | Com. Ex. 4 | Com. Ex. 5 |
|---|---|---|---|---|---|---|---|---|
| Raw materials | (A) | | UDMA | | 70 | 60 | | 45 |
| | | | AH-600 | | | | | |
| | | | U4TH | | | | | |
| | | | D2.6E | | | | | |
| | (B) | | Polyfunctional urethanized (meth)acrylic polymerizable compound (B)-I-1 | 65 | | 40 | 40 | 35 |
| | | | Polyfunctional urethanized (meth)acrylic polymerizable compound (B)-I-2 | | | | 40 | |
| | | | A-BPE-30 | | | | | |
| | (C) | | CTMPFA | 35 | 30 | | | |
| | | | MEDOMA | | | | 20 | |
| | | | A-DOG | | | | | 20 |
| | (D) | | TPO | 2.0 | 2.0 | 2.0 | 2.0 | 2.0 |
| | | | BHT | 0.5 | 0.5 | 0.5 | 0.5 | 0.5 |
| Properties | Strength | Flexural strength | (MPa) | 8.6 | 84 | Not shapeable | 12 | 53 |
| | | Flexural modulus | (MPa) | 180 | 2400 | | 250 | 1800 |
| | Toughness | Displacement of fracture point | | A | C | | A | C |
| | Water resistance | Flexural strength after immersion | (MPa) | 2.3 | 76 | | 8.4 | 42 |
| | | Percentage decrease | (%) | 73 | 9.5 | | 30 | 21 |
| | Impact resistance | Steel ball drop test | | A | C | | A | C |
| A-DOG: Dioxane glycol dicrylate (manufactured by Shin-Nakamura Chemical Co., Ltd.; Tg > 100°C) | | | | | | | | |

As shown in Tables 1 and 2, the shaped articles of the stereolithographic resin compositions of Examples 1 to 9 exhibited superior strength, toughness, water resistance, and impact resistance, with all components working together. The strength and water resistance of the shaped articles of the stereolithographic resin compositions of Examples 1 to 9 were particularly superior compared to the stereolithographic resin compositions of Comparative Examples 1 and 4.

The toughness of the shaped articles of the stereolithographic resin compositions of Examples 1 to 9 was superior to that of the shaped articles of the resin compositions of Comparative Examples 2 and 5.

The impact resistance of the shaped articles of the stereolithographic resin compositions of Examples 1 to 9 was superior to that of the resin compositions of Comparative Examples 2 and 5.

From the contrast between Example 1 and Comparative Examples 1 and 4, it was confirmed that the stereolithographic resin compositions cannot achieve sufficient strength with the combinations of components lacking polyfunctional (meth)acrylic polymerizable compound (A).

From the contrast between Example 1 and Comparative Example 2, it was also confirmed that the stereolithographic resin compositions cannot achieve both toughness and impact resistance with the combination of components lacking polyfunctional (meth)acrylic polymerizable compound (B).

From Comparative Example 3, it was found that a resin composition cannot be shaped when it does not contain monofunctional (meth)acrylic polymerizable compound (C).

As can be seen in Comparative Example 5, it was confirmed that the sufficient level of toughness, water resistance, and impact resistance assuming intraoral applications cannot be achieved with the combination of components using the dioxane di(meth)acrylate described in Patent Literature 1.

INDUSTRIAL APPLICABILITY

A stereolithographic resin composition of the present invention exhibits excellent strength, toughness, water resistance, and impact resistance in the shaped article, and is suited for intraoral applications such as a variety of dental materials (particularly, dental occlusal splints, and denture base materials) or various materials for treating sleep disorder (particularly, appliances for treating sleep apnea), with optimum suitability for orthodontic mouthpieces, and soft dental occlusal splints used in applications such as mouthguards used in sports.

**Claims**

1. A stereolithographic resin composition comprising a polyfunctional (meth)acrylic polymerizable compound (A) with a molecular weight of less than 1,000, a polyfunctional (meth)acrylic polymerizable compound (B) with a molecular weight of 1,000 or more, a monofunctional (meth)acrylic polymerizable compound (C) that does not comprise a hydroxyl group and comprises a cyclic ether structure, and a photopolymerization initiator (D).

2. The stereolithographic resin composition according to claim 1, wherein the polyfunctional (meth)acrylic polymerizable compound (A) is a (meth)acrylate that contains no polymer structure within a single molecule.

3. The stereolithographic resin composition according to claim 1 or 2, wherein the polyfunctional (meth)acrylic polymerizable compound (A) comprises a polyfunctional urethanized (meth)acrylic polymerizable compound (A)-I containing a urethane bond.

4. The stereolithographic resin composition according to claim 1 or 2, wherein the polyfunctional (meth)acrylic polymerizable compound (B) comprises a polyfunctional urethanized (meth)acrylic polymerizable compound (B)-I containing a urethane bond.

5. The stereolithographic resin composition according to claim 4, wherein the polyfunctional urethanized (meth)acrylic polymerizable compound (B)-I is a (meth)acrylate that comprises, within a single molecule, at least one structure selected from the group consisting of a polyester, a polycarbonate, a polyurethane, a polyether, a poly-conjugated diene, and a hydrogenated poly-conjugated diene.

6. The stereolithographic resin composition according to claim 5, wherein the polyfunctional urethanized (meth)acrylic polymerizable compound (B)-I is a (meth)acrylate that comprises, within a single molecule, at least one polyol moiety selected from the group consisting of a polyester, a polycarbonate, a polyurethane, a polyether, a poly-conjugated diene, and a hydrogenated poly-conjugated diene each having a structure derived from a C4 to C18 aliphatic chain diol unit having a branched structure.

7. The stereolithographic resin composition according to claim 1 or 2, wherein the cyclic ether structure of the monofunctional (meth)acrylic polymerizable compound (C) is a dioxane.

8. The stereolithographic resin composition according to claim 1 or 2, wherein the monofunctional (meth)acrylic polymerizable compound (C) is a mono(meth)acrylate.

9. The stereolithographic resin composition according to claim 1 or 2, wherein the monofunctional (meth)acrylic polymerizable compound (C) is at least one selected from the group consisting of (2-methyl-2-ethyl-1,3-dioxolan-4-yl)methyl (meth)acrylate, (2-methyl-1,3-dioxolan-4-yl)methyl (meth)acrylate, (2-ethyl-1,3-dioxolan-4-yl)methyl (meth)acrylate, 2-((2-methyl-1,3-dioxolan-4-yl)methoxy)ethyl (meth)acrylate, 2-((2-ethyl-1,3-dioxolan-4-yl)methoxy)ethyl (meth)acrylate, and cyclic trimethylolpropane formal (meth)acrylate.

10. A dental material comprising a shaped article of the stereolithographic resin composition of claim 1 or 2.

11. A denture base material comprising a shaped article of the stereolithographic resin composition of claim 1 or 2.

12. A dental occlusal splint comprising a shaped article of the stereolithographic resin composition of claim 1 or 2.

13. A material for treating sleep disorder, comprising a shaped article of the stereolithographic resin composition of claim 1 or 2.

14. A method for stereolithographically producing a three-dimensional shaped article with the stereolithographic resin composition of claim 1 or 2.

## INTERNATIONAL SEARCH REPORT

| International application No. |
|---|
| **PCT/JP2024/019910** |

**A. CLASSIFICATION OF SUBJECT MATTER**

*C08F 290/06*(2006.01)i; *A61C 13/15*(2006.01)i; *B29C 64/135*(2017.01)i; *B29C 64/314*(2017.01)i; *B33Y 10/00*(2015.01)i; *B33Y 70/00*(2020.01)i; *B33Y 80/00*(2015.01)i

FI: C08F290/06; B33Y70/00; B33Y80/00; B33Y10/00; B29C64/314; B29C64/135; A61C13/15

According to International Patent Classification (IPC) or to both national classification and IPC

**B. FIELDS SEARCHED**

Minimum documentation searched (classification system followed by classification symbols)

C08F290/06; A61C13/15; B29C64/135; B29C64/314; B33Y10/00; B33Y70/00; B33Y80/00

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| X | JP 2019-526660 A (ARKEMA FRANCE) 19 September 2019 (2019-09-19) claims, paragraphs [0001], [0110]-[0116], [0124], table 1, example 2 | 1-9, 14 |
| Y | claims, paragraphs [0001], [0110]-[0116], [0124], table 1, example 2 | 10-13 |
| X | WO 2017/222025 A1 (MAXELL HOLDINGS, LTD.) 28 December 2017 (2017-12-28) claims, paragraphs [0002], [0075], [0076], table 1, examples 7, 8 | 1-9, 14 |
| Y | claims, paragraphs [0002], [0075], [0076], table 1, examples 7, 8 | 10-13 |
| Y | WO 2020/129736 A1 (KURARAY NORITAKE DENTAL INC.) 25 June 2020 (2020-06-25) claims 13-18, paragraph [0001] | 10-13 |
| A | entire text | 1-9, 14 |
| Y | WO 2021/162007 A1 (KURARAY NORITAKE DENTAL INC.) 19 August 2021 (2021-08-19) claims 19-23, paragraph [0001] | 10-13 |
| A | entire text | 1-9, 14 |

☑ Further documents are listed in the continuation of Box C.   ☑ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **24 July 2024** | **06 August 2024** |

| Name and mailing address of the ISA/JP | Authorized officer |
|---|---|
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (July 2022)

## INTERNATIONAL SEARCH REPORT

<table>
<tr><td colspan="2">International application No.</td></tr>
<tr><td colspan="2">PCT/JP2024/019910</td></tr>
</table>

### C. DOCUMENTS CONSIDERED TO BE RELEVANT

| Category* | Citation of document, with indication, where appropriate, of the relevant passages | Relevant to claim No. |
|---|---|---|
| A | JP 2020-100790 A (FUJI XEROX CO., LTD.) 02 July 2020 (2020-07-02)<br>entire text | 1-14 |
| A | JP 2020-200437 A (CANON KABUSHIKI KAISHA) 17 December 2020 (2020-12-17)<br>entire text | 1-14 |

Form PCT/ISA/210 (second sheet) (July 2022)

## INTERNATIONAL SEARCH REPORT
### Information on patent family members

International application No.

**PCT/JP2024/019910**

| Patent document cited in search report | | | Publication date (day/month/year) | Patent family member(s) | | | Publication date (day/month/year) |
|---|---|---|---|---|---|---|---|
| JP | 2019-526660 | A | 19 September 2019 | WO | 2018/028903 | A1 | |
| | | | | claims, p. 29, line 20 to p. 30, line 2, table 1, example 2 | | | |
| | | | | CN | 109562596 | A | |
| WO | 2017/222025 | A1 | 28 December 2017 | US | 2019/0241691 | A1 | |
| | | | | claims, paragraphs [0002], [0085]-[0094], table1, examples 7, 8 | | | |
| | | | | EP | 3476874 | A1 | |
| | | | | CN | 109328201 | A | |
| WO | 2020/129736 | A1 | 25 June 2020 | US | 2022/0041777 | A1 | |
| | | | | claims 13-18, paragraph [0001] | | | |
| | | | | EP | 3900693 | A1 | |
| | | | | CN | 113226247 | A | |
| WO | 2021/162007 | A1 | 19 August 2021 | US | 2023/0091071 | A1 | |
| | | | | claims 19-23, paragraph [0001] | | | |
| | | | | EP | 4105250 | A1 | |
| | | | | CN | 115038730 | A | |
| JP | 2020-100790 | A | 02 July 2020 | (Family: none) | | | |
| JP | 2020-200437 | A | 17 December 2020 | US | 2021/0230341 | A1 | |
| | | | | entire text | | | |
| | | | | EP | 3872105 | A1 | |
| | | | | CN | 112888719 | A | |

Form PCT/ISA/210 (patent family annex) (July 2022)

**REFERENCES CITED IN THE DESCRIPTION**

**Patent documents cited in the description**

- WO 2020136919 A
- JP 2000159621 A